(19) Europäisches Patentamt
European Patent Office
Office européen des brevets

(11) **EP 1 842 548 A1**

(12) **EUROPEAN PATENT APPLICATION**
published in accordance with Art. 158(3) EPC


(43) Date of publication:
**10.10.2007 Bulletin 2007/41**

(21) Application number: **05811568.4**

(22) Date of filing: **30.11.2005**

(51) Int Cl.:
***A61K 38/00*** (2006.01) ***A61P 31/18*** (2006.01)
***C12P 21/02*** (2006.01) ***C12N 1/15*** (2006.01)
***C12N 1/21*** (2006.01) ***C12N 15/09*** (2006.01)
***C07K 14/36*** (2006.01)

(86) International application number:
**PCT/JP2005/021981**

(87) International publication number:
**WO 2006/059638 (08.06.2006 Gazette 2006/23)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.11.2004 JP 2004346644**



(71) Applicant: **THE KITASATO GAKUEN FOUNDATION**
**Tokyo 1088641 (JP)**

(72) Inventors:
- **TANAKA, Haruo,**
**THE KITASATO GAKUEN FOUNDATION**
**Kanagawa 228-8555 (JP)**
- **INOKOSHI, Junji,**
**THE KITASATO GAKUEN FOUNDATION**
**Kanagawa 228-8555 (JP)**
- **OMURA, Satoshi,**
**THE KITASATO GAKUEN FOUNDATION**
**Kanagawa 228-8555 (JP)**

(74) Representative: **Horner, Martin Grenville et al**
**Marks & Clerk**
**19 Royal Exchange Square**
**GB-Glasgow G1 3AE (GB)**


(54) **ANTI-HIV DRUG, POLYPEPTIDE CONSTITUTING THE SAME, GENE ENCODING THE POLYPEPTIDE AND METHOD OF PRODUCING THE ANTI-HIV DRUG**


(57) It is intended to provide an anti-HIV agent **characterized by** comprising a multimer of Actinohivin; a polypeptide which is a multimer of Actinohivin; constituting the same, DNA gene encoding the polypeptide; and a method for producing the anti-HIV agent. The present invention is aimed to provide the anti-HIV agent working by inhibiting syncytium formation and having enhanced effect.


Fig.1 Construction method of the actinohivin dimer

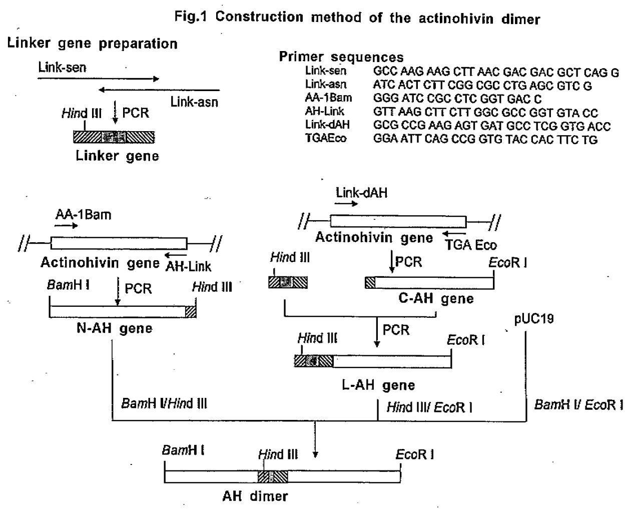


EP 1 842 548 A1

## Description

FIELD OF THE INVENTION

The present invention relates to an anti-viral agent for human immunodeficiency virus (HIV), i.e., an anti-HIV agent, a polypeptide constituting the same, DNA encoding the polypeptide, a transformant transformed with the DNA, and a method for producing the anti-HIV agent. More specifically, the present invention relates to an anti-viral agent for HIV-1 (an anti-HIV agent) , a polypeptide constituting the same, DNA encoding the polypeptide, a transformant transformed with the DNA, and a method for producing the anti-HIV agent.

BACKGROUND ART

Currently, reverse transcriptase inhibitors and protease inhibitors are used as therapeutic agents for treating acquired immunodeficiency syndrome (AIDS) caused by HIV.

Reverse transcriptase inhibitors are classified into two major groups: nucleotide-type inhibitors and non nucleotide-type inhibitors. It is known that prolonged administration of nucleotide-type inhibitors will cause severe side effects, and that resistant strains will emerge after about one year from the initial administration of the inhibitors. Non nucleotide-type inhibitors will cause fewer side effects, but due to their highly specific action, resistant strains will emerge early on. On the other hand, protease inhibitors show good anti-viral activities even when administered alone, but they will generally be effective only transiently, and the sensitivities will decline due to the mutations of the amino acid sequences of HIV proteases. In addition, they involve problems of low stabilities *in vivo,* side effects such as the digestive system disorder and so on.

The infection of HIV is established by its adhering and entering to a host cell via the binding between an HIV surface protein, glycoprotein 120 (gp120), and a surface receptor of the host cell, CD4. It is expected that a substance that inhibits the binding of gp120 and CD4 will inhibit the adhesion of HIV and the cell, and thus it will prevent HIV infection. Thus, attempts have been made to inhibit the binding between gp120 and CD4. For example, attempts such as the administration of soluble CD4 prepared by genetic engineering to human subjects have been done. However, by such a method, an anti-HIV activity was not observed due to the reasons such as short half-lives *in vivo* even though the inhibitory activity had been recognized *in vitro.* Further, it was revealed that murine cells carrying expressed human CD4 could not be infected, suggesting the presence of a second adhesion molecule.

Recently, it was repeatedly reported that the second receptor was a member of chemokine receptors. HIV strains are divided into two major groups, i.e., one containing macrophage-tropic strains (M-tropic HIV) and the other containing T cell-tropic strains (T-tropic HIV). It was suggested that the difference in cell tropism observed between these groups of viral strains was due to the differences in the molecular species of the second receptor. That is, it was demonstrated that the cell tropism of HIV strains was determined by the type of the second receptor molecule, either CC chemokine receptor 5 (CCR5: M-tropic HIV receptor) or CXC chemokine receptor 4 (CXCR4: T-tropic HIV receptor), expressed on the target cells. Based on these new findings, the manner of entry of HIV into the target cells is currently hypothesized as follows. First, the binding of gp120 and CD4 occurs, and then the resultant binds with CCR5 or CXCR4 expressed on the host cells. As a result, gp120 is structurally altered to expose glycoprotein 41 (gp41), which in turn binds to the cell membrane to establish the infection. On the other hand, corresponding chemokines competitively block the binding of HIV to the chemokine receptors to suppress the HIV infection. The series of findings not only facilitated elucidation of the mechanisms of infection and disease development, but also provided a new viewpoint for anti-HIV strategies.

From such a viewpoint, the present inventors searched metabolites produced by microorganisms, and have found that the culture fluid of a ray fungus strain K97-0003 (deposited on Dec. 9, 1998 to International Patent Organism Depositary (IPOD) , National Institute of Advanced Industrial Science and Technology (AIST), Central 6, 1-1 Higashi, Tsukuba, Ibaraki, 305-8566 Japan, under the deposition number FERM BP-6670) has a superior HIV-inhibitory activity and have shown that the active component is a substance that inhibits the syncytium formation of the mechanisms as outlined above (Patent Document 1: International Publication WO11/52043 pamphlet).

Patent Document 1: International Publication WO11/52043 pamphlet

DISCLOSURE OF THE INVENTION

PROBLEM TO BE SOLVED BY THE INVENTION

The present invention is aimed to provide an anti-HIV agent working by inhibiting syncytium formation and having enhanced effects compared to the polypeptide as disclosed in WO00/52043.

MEANS FOR SOLVING THE PROBLEM

The present inventors further investigated for the polypeptide having 114 amino acid residues as disclosed in WO00/52043 (SEQ ID No. 1) . As a result, the present inventors have found that a multimer of the polypeptide has greater anti-HIV effects, and completed the present invention.

The present invention provides the following anti-HIV agent(s), polypeptide (s) , base sequence(s), transformant (s), and a method for producing the anti-HIV agent(s).

(1) An anti-HIV (human immunodeficiency virus) agent comprising a multimer of actinohivin.
(2) The anti-HIV agent according to (1) above, wherein the multimer of actinohivin is a multimer in which two or more polypeptides selected from:

(a) the polypeptide as defined in SEQ ID No. 1; and
(b) a homologue of (a) above having inhibitory activity on syncytium formation, in which one or more amino acids in the amino acid sequence of (a) have been deleted, substituted or added (the polypeptides may be the same or different) are linked with a linker consisting of up to fifty amino acids.

(3) The anti-HIV agent according to (1) or (2) above, wherein the multimer of actinohivin is a dimer of actinohivin.
(4) The anti-HIV agent according to any one of (1) to (3) above, wherein the linker is a peptide chain as defined in SEQ ID No. 2.
(5) The anti-HIV agent according to any one of (1) to (4) above, further comprising a His tag.
(6) A polypeptide corresponding to the anti-HIV agent according to any one of (1) to (5) above.
(7) A base sequence encoding the polypeptide according to (6) above or a complementary strand thereof.
(8) A transformant in which the base sequence according to (7) above has been introduced into a host cell.
(9) The transformant according to (8) above, wherein the host cell is E. coli or actinomycete.
(10) The transformant according to (9) above, wherein the transformant is Escherichia coli BL21(DE3) pLysS/pET30: dAH strain (International Patent Organism Depositary (IPOD), National Institute of Advanced Industrial Science and Technology (AIST) Accession No. FERM BP-10161).
(11) A method for producing the anti-HIV agent according to any one of (1) to (5) above, comprising the steps of culturing the transformant according to (9) or (10) above to produce in the transformant cells or in the culture the polypeptide according to (6) above, and isolating the same.

PREFERRED EMBODIMENTS OF THE INVENTION

ANTI-HIV AGENT

In a first embodiment of the invention, an anti-HIV agent comprising a multimer of actinohivin is provided. Actinohivin herein means:

(a) the polypeptide as defined in SEQ ID No. 1; and
(b) a homologue having inhibitory activity on syncytium formation, in which one or more amino acids in the amino acid sequence of (a) above have been deleted, substituted or added.

The polypeptide in which a part of the amino acid sequence has been deleted, substituted or added, as stated in (b) above means a polypeptide having at least 20%, preferably 30% or more, more preferably 50% or more, further more preferably 80% or more, and most preferably 90% or more of amino acid homology with the amino acid sequence as defined in SEQ ID No. 1. As long as the inhibitory activity on syncytium formation is exhibited, the deletion, substitution or addition of an amino acid may occur at any position.

The polypeptide as defined by SEQ TD No. 1 is constituted from three homologous domains (in SEQ ID No. 1, 1-38, 39-76 and 77-114). Those actinohivin multimer (n-mer) with one or two homologous domains added are also included. The number (n) of the actinohivin unit contained in an actinohivin multimer (n-mer) is not restricted as long as the inhibitory activity on syncytium formation is exhibited. Considering the handling in the purification process and so on, normally hexamer or less, preferably trimer or less, e.g., dimer is used.

The inhibitory activity on syncytium formation may be suitably evaluated. For example, evaluation can be conducted based on the presence or absence of fusion of: (a) cells expressing envelope glycoprotein (gp120, gp41) (e.g., HeLa cells) and (β) cells expressing the genes of an auxiliary receptor (CXCR4 or CCR5) of HIV-1 that is essential for the entry of HIV-1 and CD4 (e.g., HeLa cells). In practice, those cells to which a transcription activation protein (Tat) gene has been further introduced and those to which HIV LTR (long terminal repeat sequence) and β-galactosidase gene has been further introduced are used as the (α) and (β), respectively.
By using such cells, the presence or absence of syncytium formation (HIV infection) can be judged by color development from X-gal (5-bromo-4-chloro-3-indoryl-beta-galactoside), the substrate for β -galactosidase, because β -galactosidase is activated along with the HIV infection of the cells.

In the context of the present invention, an actinohivin multimer means the amino acid sequences as defined in (a) or (b) above which have been linked each other or one another either directly or via any linker sequence, and may contain a sequence useful for purification or handling of the polypeptide.

The linker sequence is not limited as long as it does not disturb the anti-HIV action of the multimer. It is a sequence consisting of generally fifty or less, preferably 20 or less, more preferably 15 or less amino acid residues. For example, a polypeptide as defined in SEQ ID No. 2 is among those linkers. Accordingly, the actinohivin multimer of the invention includes a polypeptide consisting of the polypeptide of SEQ ID No. 1 - the linker sequence of SEQ ID No. 2 - the polypeptide of SEQ ID No. (indicated as SEQ ID No. 3). This polypeptide is a dimer. Similar structures of trimer or more are possible.

Examples of those additional sequences useful for the purification or handling of the polypeptide include His tags. GST tags may be used as well, but generally these modification sequences tend to decrease the anti-HIV action. An example of His tags is $His_6$ tag.

The reason why actinohivin multimers exert excellent activities is not clear. However, it has been demonstrated that actinohivin binds the high-mannose sugar chain of gp120 and thus it is possible that binding as a cluster enhances the anti-HIV effect.

POLYPEPTIDE

In a second embodiment of the present invention, a polypeptide corresponding to said anti-HIV agent comprising an actinohivin multimer is provided. The details of such a polypeptide are similar to those described as the anti-HIV agent.

BASE SEQUENCE

In a third embodiment of the present invention, a base sequence coding for said polypeptide or a complementary strand thereof is provided.
For example, the base sequences provided by the present invention include the base sequence of SEQ ID No. 4 (including a stop codon) corresponding to the polypeptide of SEQ ID No. 3. More generally, base sequences of two or more actinohivin coding regions selected from:

(a') a base sequence coding for the polypeptide indicated by SEQ ID No. 1; and
(b') a base sequence coding for a homologue having inhibitory activity on syncytium formation and having an amino acid sequence in which one or more amino acids have been deleted, substituted, or added in the amino acid sequence of (a'),

linked each other via a base sequence coding for a linker sequence, are provided.

As used herein, the meanings of the terms "a homologue", "a linker sequence", and "inhibitory activity on syncytium formation" are as defined above. For example, a homologue has at least 20%, preferably 30% or more, more preferably 50% or more, further more preferably 80% or more, and most preferably 90% or more of homology with the base sequence coding for the amino acid sequence as defined in SEQ ID No. 1. As stated above, the actinohivin monomer consists of three homologous domains, and a homologue may contain a base sequence corresponding to one or two homologous domains in addition to a base sequence corresponding to a multimer. The number (n) of actinohivin coding regions contained in a base sequence is not limited as long as the actinohivin multimer eventually obtained retains inhibitory activity on syncytium formation. However, considering the handling during purification and so on as described above, it is normally six or less, preferably three or less, namely two.

In addition, the base sequence of the present invention may contain a sequence useful for the production of the polypeptide. For example, when an actinohivin multimer is expressed in vivo, if it is necessary or beneficial to produce a precursor polypeptide first, and then modify it after translation to obtain the actinohivin multimer as a mature protein, a base sequence for doing so may be contained. Those base sequences include a signal peptide sequence region used for the secretion outside from a microorganism. Further, a sequence useful for the purification or handling of the polypeptide may also be contained. Those base sequences include a base sequence corresponding to a His tag.

In the present application, a base sequence coding for a polypeptide may be coded by any of those degenerate codons. However, base sequences suitable for the expression in a transformant which is described below are preferred. Base sequences may also be a complementary strand of the above described sequences.

TRANSFORMANT

In a fourth embodiment of the present invention, a transformant in which the above described base sequence has been introduced into a host cell is provided. The transformant can be anything, as long as it can stably express an actinohivin multimer, and includes E. coli, actinomycete, other bacteria, fungi, yeasts, animal cells, insect cells and so on. Further, cells which have been mutated by cell engineering using techniques such as cell fusion and genetic manipulation are also included in hosts of the transformant of the present invention.

Particularly preferred host cells are E. coli and actinomycete. Examples of those transformants include *Escherichia* coli BL21 (DE3) pLysS/pET30:dAH strain (deposited on November 10, 2004 with International Patent Organism Depositary (IPOD), National Institute of Advanced Industrial Science and Technology (AIST), Central 6, 1-1 Higashi, Tsukuba, Ibaraki, 305-8566 Japan, under the Accession number FERM BP-10161).

The transformant may be produced using a method known in the relevant art (see, for example, Joseph Sambrook et al., "Molecular Cloning", 3rd Ed. (Cold Spring Harbor Laboratory Press(2001)).

PROCESS FOR PRODUCING THE ANTI-HIV AGENT

In the fourth embodiment of the present invention, a process for producing an actinohivin multimer anti-HIV agent comprising culturing the transformant to cause the production of said polypeptide in the transformant or in the culture and isolating the product.

For culturing the transformant of the present invention, a conventional method normally utilized for culturing such transformant can be adopted. As for the culture medium, either a natural medium or a synthetic medium can be used, as long as it contains a carbon source catabolizable for the transformant, a nitrogen source, an inorganic substance, and so on in proper amounts.

As a carbon source, carbohydrates, such as glucose, mannose, maltose and molasses; organic acids, such as citric acid, malic acid, acetic acid and fumaric acid; alcohols, such as methanol and ethanol; hydrocarbons, such as methane, ethane, propane and n-paraffin; amino acids, such as glutamic acid; or glycerol and so on may be used.

As a nitrogen source, ammonium salts, such as ammonium chloride, ammonium sulfate, ammonium nitrate and ammonium phosphate; amino acids, such as aspartic acid, glutamine, cystine and alanine; urea, peptone, meat extract, yeast extract, dry yeasts, cone steep liquor, soybean flour, soluble vegetable protein, cottonseed oil, soybean casein, casamino acids, Pharmamedia, and so on may be used. As an inorganic substance, potassium monohydrogenphosphate, potassium dihydrogenphosphate, sodium dihydrogenphosphate, magnesium phosphate, magnesium sulfate, ferrous sulfate, manganese sulfate, copper sulfate, cobalt sulfate, zinc sulfate, calcium pantothenate, ammonium molybdate, aluminum potassium sulfate, barium carbonate, calcium carbonate, cobaltous chloride, salt, and so on may be used. In addition, substances for promoting the proliferation of the cells or the production of an actinohivin multimer, such as a small amount of metal salts, vitamins, thiamin, and so on may be added to the medium if needed. Furthermore, when a specific substance is required for the growth of the transformant, it is necessary to add such required substance. These substances may be any of those useful for the production of an actinohivin multimer. For example, all the materials known as being useful for culturing a transformant, particularly E. coli or actinomycetes, can be used.

For large scale culture of a transformant, especially a microorganism, those culture methods that are performed in a liquid medium with shaking, with aerating and stirring, and so on are preferable. The temperature of culturing may be selected within the range in which the transformant can grow and produce an actinohivin multimer. Culturing may be appropriately carried out depending on the nature of the transformant. When the actinohivin multimer is present in the culture fluid, it may be possible to recover the culture fluid containing the cells and to use it per se. However, generally an actinohivin multimer solution is used after the actinohivin multimer and the transformant in the culture fluid have been separated according to a conventional method such as filtration and centrifugation. When the actinohivin multimer is present in the transformant cells, the cells may be recovered from the culture by means of filtration, centrifugation and so on, then disrupted by a mechanical method or an enzymatic method such as the use of lysozyme, and optionally lysed by adding a chelating agent such as EDTA and/or a surfactant when necessary, to separate and obtain the multimer in a form of an aqueous solution.

The solution containing the actinohivin multimer thus obtained can be concentrated by, for example, reduced pressure concentration or membrane concentration, and further precipitated by salting-out using ammonium sulfate or sodium sulfate, or by fractional precipitation using a hydrophilic organic solvent such as methanol, ethanol, acetone, and so on. Then, the precipitate may be solved in water and dialysed with a semi-permeable membrane to remove low molecular weight impurities. Gel filtration with an absorbent or a gel filtration matrix, adsorption chromatography, ion exchange chromatography or reversed phase chromatography may also be utilized for purification. In particular, in the embodiment in which actinohivin multimer containing a His tag is produced, it can be purified by a column with a solid phase having high affinity to the tag, such as Ni-, Zn-, Cu-, or Co-column.

The actinohivin multimer-containing solution obtained by these means can be further subjected to vacuum concentration, lyophilization, and so on to obtain purified actinohivin multimer. The peptide added to the actinohivin multimer, e.g., a His tag, may be further removed as necessary.

## EXAMPLES

The present invention is further explained more specifically below by means of examples. However, these examples are not intended to limit or restrict the scope of the present invention.

## EXAMPLE 1

### (1) Construction of gene coding for actinohivin dimer

The gene (SEQ ID No. 4) coding for the actinohivin dimer as defined in SEQ ID No. 3 was constructed according to the protocol as shown in Figure 1.

Firstly, a linker gene coding for 13 amino acid residues for ligating two actinohivin genes was prepared as follows.

Synthetic oligonucleotide Link-sen (SEQ ID No. 5) and Link-asn (SEQ ID No. 6) were annealed at the homologous sequence sites of 11 bases added to the 3'- and 5'-termini, respectively, of the both primers to form a primer dimer. The resultant was subjected to PCR under the conditions of 30 seconds at 94°C, 60 seconds at 60°C, and 1 minute at 72°C (30 cycles).

Next, an actinohivin gene corresponding to the N-terminal portion of actinohivin dimer (N-AH gene) was prepared by PCR under the conditions of 30 seconds at 94°C, 30 seconds at 60°C, and 1 minute at 72°C (30 cycles) using two synthetic oligonucleotide primers AA-1Bam (SEQ ID No. 7) and AH-Link (SEQ ID No. 8) , and plasmid 2A3K carrying AH gene cloned from an actinohivin-producing microorganism as the template. The resulting N-AH gene has the addition of 15 base pairs of the 5'-terminal portion of the linker gene at the 3'-terminus of actinohivin gene.

An actinohivin gene corresponding to the C-terminal portion of actinohivin dimer (C-AH gene) was prepared as follows. PCRwas conducted under the conditions of 30 seconds at 94°C, 30 seconds at 60°C, and 1 minute at 72°C (30 cycles) using Link-dAH (SEQ ID No. 9) and TGAEco (SEQ ID No. 10), and plasmid 2A3K as the template. The resulting C-AH gene has the addition of 15 bases of the 3'-terminal portion of the linker gene at the 5'-terminus of actinohivin gene.

L-AH gene, which had the linker gene added at the 5'-terminus of actinohivin gene, was prepared by PCR using the linker gene and C-AH gene as the template and the synthetic oligonucleotides Link-sen and TGAEco as described above, according to the SOE method (Horton, R.M., Hunt, H.D., Ho, S.N., Pullen, J.K. and Pease, L.R. Engineering hybrid genes without the use of restriction enzymes: gene splicing by overlap extension. Gene 77, 61~68 (1989)).

The thus obtained N-AH gene and L-AH gene were digested with restriction enzymes BamH I and Hind III, and Hind III and EcoR I, respectively. The resulting two DNA fragments were mixed with E. coli cloning vector pCTC19 that had been digested with BamH I and EcoR I, and ligated using T4 DNA ligase. E. coli strain JM109 was transformed with the resulting solution to obtain plasmid pUC19:dAH containing actinohivin dimer gene.

### (2) Construction of actinohivin dimer expression plasmid

An actinohivin dimer gene was amplified by PCR using pUC19:dAH as the template and AA-1 LIC (SEQ ID No. 11) and TGA 114 LIC (SEQ ID No. 12) under the conditions of 30 seconds at 94°C, 30 seconds at 60°C, and 1 minute at 72°C (30 cycles). Resulting DNA fragments were subjected to T4 DNA polymerase reaction in the presence of dGTP, followed by ligation with E. coli expression vector pET30 Xa/LIC, to construct the actinohivin dimer expression plasmid pET30:dAH.

### (3) Expression of actinohivin dimer by E. coli

LA medium used was containing kanamycin sulfate (KM) and chloramphenicol (CM) added so as to make the final concentrations of 30$\mu$g/ml and 34$\mu$g/ml, respectively.
A colony of E. coli BL21 (DE3)plysS transformed with the actinohivin dimer expression plasmid pET30: dAH (deposited on November 10, 2004 with International Patent Organism Depositary (IPOD), National Institute of Advanced Industrial Science and Technology (AIST) (Central 6, 1-1 Higashi, Tsukuba, Ibaraki, 305-8566 Japan), under the Accession number FERM BP-10161) formed on LA medium was used to inoculate 1ml of LB medium containing 2% glucose, KM (30 $\mu$g/ml) and CM (34$\mu$g/ml) and cultured at 37°C overnight with shaking. Eight ml of this culture was added to 800 ml of LB medium containing KM (30 $\mu$g/ml) and CM (34$\mu$g/ml) and cultured at 37°C with shaking. Isopropyl- β -thiogalactopyranoside (IPTG) was added at $OD_{600nm}$ of 0.3, and the culture was continued further two hours at 37°C with shaking.

After the growth of E. coli was stopped by quenching the medium with iced water, the culture fluid was centrifuged at 12,000×g for 15 minutes at 4°C to recover the cells. The obtained cells were washed twice with PBS(-) and stored at -20°C.

COMPARABLE EXAMPLE 1

(1) Construction of actinohivin (monomer) expression plasmid

Actinohivin gene as defined in SEQ ID No. 1 was amplified by performing PCR using plasmid 2A3K as the template and AA-1 LIC (SEQ ID No. 11) and TGA 114 LIC (SEQ ID No. 12) as described above under the conditions of 30 seconds at 94°C, 30 seconds at 60°C, and 1 minute at 72°C (30 cycles). The resulting DNA fragment was subjected to T4DNA polymerase reaction in the presence of dGTP and then ligated to E. coli expression vector pET30 Xa/LIC to constract the actinohivin (monomer) expression plasmid pET30:AH.

(2) Expression of actinohivin (monomer) by E. coli

Actinohivin (monomer) was expressed in E. coli BL21 (DE3)plysS transformed by a procedure similar to that in Example 1, (3), except for that the actinohivin (monomer) expression plasmid pET30:AH was used in place of the actinohivin dimer expression plasmid pET30:dAH.

EXAMPLE 2

According to the procedures described below, the actinohivin fusion protein was prepared and purified, and tested for its anti-HIV effects.

(1) Preparation of E. coli extract

According as Example 1, E. coli cells containing the actinohivin dimer with a His tag expressed in the cells were suspended in 10 ml of a binding buffer (5 mM imidazole, 0.5 M NaCl, 20 mM Tris-HCl (pH 7.9)). The cells were disrupted by sonication of 1 minute with 30 seconds interval (total 5 minutes). The resulting E. coli extract was centrifuged at 8, 370×g for 10 minutes at 4°C and the supernatant and pellet were separated. The pellet obtained was dissolved in the binding buffer containing 6 M guanidine (hereinafter, the binding buffer DN), and the solution was centrifuged at 8,370xg for 10 minutes at 4°C to remove the pellet.

(2) Affinity chromatography with metal chelate Sepharose 4B

Five ml of metal chelate Sepharose 4B was poured in a 15 ml plastic tube, to which 10 ml of milliQ water was added. After gently stirred, the solution was centrifuged at 264×g for 3 minutes at room temperature to remove the supernatant. Five ml of a charge buffer (50 mM $NiSO_4$) was added and the solution was stirred to activate the resin. After being washed twice with 10 ml of milliQ water, the resin was equilibrated with 10 ml of the binding buffer (DN).

Five ml of activated resin and the sample prepared in (1) above were mixed in a 50 ml plastic tube. After the total volume was adjusted to 50 ml with the binding buffer (DN), the mixture was gently shaken for 1 hour at room temperature. The resin was washed with 10 ml of the binding buffer (DN), then filled in a column, and washed with 50 ml of the binding buffer (DN). Next, the resin was washed with 50 ml of a washing buffer (60 mM imidazole, 0.5 M NaCl, 20 mM Tris-HCl (pH 7.9)) containing 6 M guanidine hydrochloride, and then the His-tagged actinohivin dimer fusion protein adsorbed to the resin was eluted with 50 ml of an elution buffer (500 mM imidazole, 0.5 M NaCl, 20 mM Tris-HCl (pH 7.9)) containing 6 M guanidine hydrochloride.

(3) ODS Column chromatography

Ten ml of ODS resin suspended in acetonitrile was filled in a column, and the column was washed with 50 ml of 1:1 mixture of acetonitrile : 0.01% trifluoro acetic acid (TFA) and then equilibrated with 50 ml of 5:95 mixture of acetonitrile : 0.01% TFA. The His-tagged actinohivin dimer fusion protein purified by metal chelate Sepharose 4B was loaded on the top of the column. After the column was washed with 50 ml of 5:95 mixture of acetonitrile : 0.01% TFA, the His-tagged actinohivin dimer fusion protein adsorbed to the column was eluted with 50 ml of 1:1 mixture of acetonitrile : 0.01% trifluoro acetic acid (TFA). The resulting fluent was concentrated, dried and then dissolved in MilliQ water.

(4) HPLC using ODS column

The sample solution desalted with the ODS column was dissolved in a small quantity of MilliQ water, subjected to high performance liquid chromatography (PEGASIL-B, 10 ϕ × 250mm, SSC, Senshu Scientific Co., Ltd.). Using a linear gradient of 5: 95 mixture of acetonitrile : 0.01% TFA and 70:30 mixture of acetonitrile : 0.01% TFA in 30 minutes, the peaks appearing at 12 minutes and 16 minutes at the flow rate of 3 ml/min were collected with the absorption at 220 nm being monitored. Each of these fractions were repeatedly frozen and thawed until the pH became around neutral and dissolved in milliQ water.

(5) Measurement of inhibitory activity on syncytium formation

HeLa/CD4 /LTR cells adjusted to 1.6 × 105 cells/ml with DME medium were dispensed at 50 μl/well into wells of a 96-well plate, to which the sample solution serially diluted with serum-free DME medium was added at 10 μl/well. Further, HeLa135/ T-env(envelope glycoprotein derived from T-tropic HIV-1) /Tat cells adjusted to 1.6 × $10^5$ cells/ml were dispensed at 50 μl/well and the plate was incubated at 37 °C under 5% $CO_2$ atmosphere for 24 hours. The culture medium was removed and the cell lysis solution was added at 20 μl/well and the plate was kept standing for 15 minutes at room temperature. After 100 μl of chromogenic substrate solution (a mixture of 20 μl of o-nitrophenyl-β-D-glactopyranoside and 80 μl of Z-buffer (60 mM disodium hydrogenphosphate, 40 mM sodium dihydrogenphosphate, 10 mM potassium chloride, 1mM magnesium sulfate, and 50 mM 2-mercaptoethanol, pH 7.0)) was added to each well and reacted at 37 °C for 80 minutes, the reaction was stopped by adding 25 μl/well of 2 M $Na_2CO_3$, and the absorbance at 405 nm was measured using PAWER WAVE X340(BIO TEC INSTROMENTS CO.). The absorbance measurements were used to calculate the percentages of syncytium formation based on the following formula:
[Formula 1]

The percentage of syncytium formation = (the absorbance of the well with sample addition / the absorbance of the control well) × 100

The concentrations that inhibit 50% of syncytium formation by the actinohivin fusion protein ($IC_{50}$) thus obtained is shown in Table 1.
Further, by conducting an experiment similar to that described for the preparation of His-tagged the actinohivin dimer fusion protein, the His-tagged actinohivin monomer fusion protein was prepared and the percentage of syncytium formation was measured. The results are also shown in Table 1.
Table 1

| His-tagged fusion protein | concentration that inhibit 50% of syncytium formation ($IC_{50}$) |
|---|---|
| actinohivin dimer fusion protein | 0.235 |
| actinohivin monomer fusion protein | 0.58 |

Based on the results above, it is demonstrated that the actinohivin dimer fusion protein of the present invention has more than twice inhibitory activity on syncytium formation compared to that of the actinohivin monomer fusion protein.
It is noted that a His tag is known to reduce inhibitory activity on the syncytium formation to about 1/7 to 1/8 and that the concentration that inhibits 50% of syncytium formation of the actinohivin dimer fusion protein without a His tag is expected to be around 0.03 μM.

INDUSTRIAL APLICABILITY

The actinohivin multimer protein of the present invention inhibits syncytium formation which is a critical pathway in HIV infection. The working mechanism is considered to be similar to that of the inhibition of syncytium formation by actinohivin and thus it is expected to be effective in preventing infection not only by T-tropic HIV but also by M-tropic HIV. Moreover, the anti-HIV effect of actinohivin dimer protein of the present invention, for example, is more than twice of that of the actinohivin monomer fusion protein, and is remarkable with only a very small amount. Therefore, according to the present invention, an anti-HIV agent polypeptide that can be used as a therapeutic and/or preventive agent of AIDS is provided. Further, the gene coding for the actinohivin multimer protein of the present invention can be introduced into various hosts to produce a recombinant actinohivin multimer protein and it can be readily produced.

BRIEF DESCRIPTION OF THE DRAWINGS

[Figure 1] Figure 1 is a graphic of the outline of the construction method of the actinohivin dimer.

## Claims

1. An anti-HIV (human immunodeficiency virus) agent comprising a multimer of actinohivin.

2. The anti-HIV agent according to Claim 1, wherein the multimer of actinohivin is a multimer in which two or more polypeptides selected from:

   (a) the polypeptide as defined in SEQ ID No. 1; and
   (b) a homologue of (a) above having inhibitory activity on syncytium formation, in which one or more amino acids in the amino acid sequence of (a) have been deleted, substituted or added

   (the polypeptides may be the same or different) are linked with a linker consisting of up to fifty amino acids.

3. The anti-HIV agent according to Claim 1 or 2, wherein the multimer of actinohivin is a dimer of actinohivin.

4. The anti-HIV agent according to any one of Claims 1 to 3, wherein the linker is a peptide chain as defined in SEQ ID No. 2.

5. The anti-HIV agent according to any one of Claims 1 to 4, further comprising a His tag.

6. A polypeptide corresponding to the anti-HIV agent according to any one of Claims 1 to 5.

7. A base sequence encoding the polypeptide according to Claim 6 or a complementary strand thereof.

8. A transformant in which the base sequence according to Claim 7 has been introduced into a host cell.

9. The transformant according to Claim 8, wherein the host cell is E. coli or actinomycete.

10. The transformant according to Claim 9, wherein the transformant is Escherichia coli BL21 (DE3) pLysS/pET30:dAH strain (International Patent Organism Depositary (IPOD), National Institute of Advanced Industrial Science and Technology (AIST) Accession No. FERM BP-10161).

**11.** A method for producing the anti-HIV agent according to any one of Claims 1 to 5, comprising the steps of culturing the transformant according to Claim 9 or 10 to produce in the transformant cells or in the culture the polypeptide according to Claim 6, and isolating the same.

Fig.1 Construction method of the actinohivin dimer

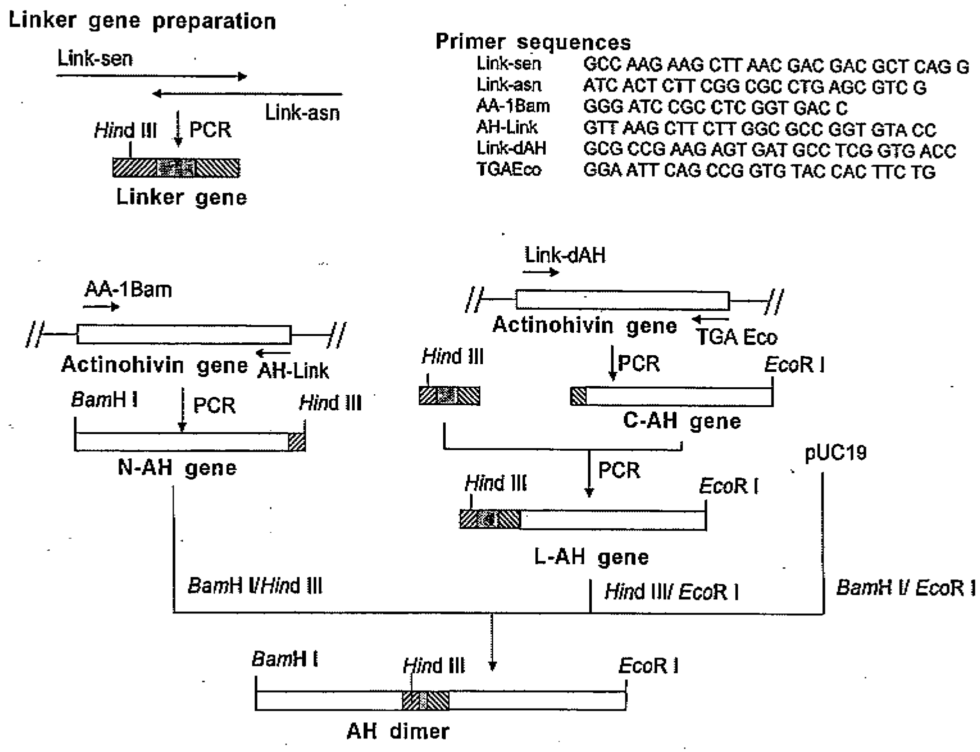

## INTERNATIONAL SEARCH REPORT

International application No.
PCT/JP2005/021981

A. CLASSIFICATION OF SUBJECT MATTER
***A61K38/00***(2006.01), ***A61P31/18***(2006.01), ***C12P21/02***(2006.01), ***C12N1/15***(2006.01), ***C12N1/21***(2006.01), ***C12N15/09***(2006.01), ***C07K14/36***(2006.01)

According to International Patent Classification (IPC) or to both national classification and IPC

B. FIELDS SEARCHED

Minimum documentation searched (classification system followed by classification symbols)
A61K38/00, A61P31/18, C07K14/36, C12N1/15, C12N1/21, C12N15/09, C12P21/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), BIOTECHABS(STN), CA(STN), EMBASE(STN), MEDLINE(STN), REGISTRY(STN), SwissProt/PIR/Geneseq, PubMed

C. DOCUMENTS CONSIDERED TO BE RELEVANT

| Category* | Citation of document, with indication, where appropriate, of the relevant passages | Relevant to claim No. |
|---|---|---|
| Y | WO 00/52043 A1 (Gakko Hojin Kitazato Gakuen), 08 September, 2000 (08.09.00), Page 13, line 1 to page 21, line 16 & AU 9957586 A & EP 1076065 A1 & JP 2000-602267 X & AU 750914 B & US 6482412 B1 | 1-11 |
| Y | CHIBA, H. et al., Actinohivin, a novel anti-HIV protein from an actinomycete that inhibits syncytium formation: Isoration, characterization, and biological activities. Biochem.Biophys.Res. Commun., 2001, Vol.282, pages 595 to 601, ISSN 0006-291X, abstract, Figs. 7, 8 | 1-11 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 January, 2006 (31.01.06) | 07 February, 2006 (07.02.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2005/021981

C (Continuation). DOCUMENTS CONSIDERED TO BE RELEVANT

| Category* | Citation of document, with indication, where appropriate, of the relevant passages | Relevant to claim No. |
|---|---|---|
| Y | CHIBA, H. et al. Actinohivin, a novel anti-human immunodeficiency virus protein from an antinomycete, inhibits viral entry to cells by binding high-mannnose type sugar chains of gp120. Biochem.Biophys.Res.Commun., 2004, Vol.316, pages 203 to 210, ISSN 0006-291X, abstract, page 204 to 206, Figs. 1, 2, 3 | 1-11 |
| Y | INOKOSHI, J. et al. Molecular cloning of Actinohivin, a novel anti-HIV protein from an actinomycete, and its expression in Escherichia coli.Biochem.Biophys.Res.Commun., 2001, Vol.281, pages 1261 to 1265, ISSN 0006-291X, abstract, materials and methods, Fig. 5 | 1-11 |
| Y | KRAJEWSKI, K. et al. Synthesis and HIV-1 integrase inhibitory activity of diametric and tetrametric analogs of indolicidin.Bioorg.Med. Chem.Lett., 2004, Vol.14, pages 5595 to 5598, ISSN 0960-894X, abstract, Table 1 | 1-11 |
| Y | KRAJEWSKI, K. et al. Design and synthesis of dimeric HIV-1 integrase inhibitory peptides. Bioorg.Med.Chem.Lett., 2003, Vol.13, pages 3203 to 3205, ISSN 0960-894X, abstract, Fig. 1, Table 1 | 1-11 |
| Y | BAARDSNES, J. et al. Antifreeze protein dimer, when two ice-binding faces are better than one. J.Biol.Chem., 2003, Vol.278, No.40, pages 38942 to 38947, abstract, page 38944 to 38945, Figs. 1, 3 | 1-11 |
| Y | WO 03/072594 A2 (Government of the united states of America), 04 September, 2003 (04.09.03), Pages 13 to 15 & US 2005/0090643 A1 & EP 1478392 A2 & AU 2003212459 A1 | 1-11 |
| Y | CARRITHERS, M. et al. Synthesis and characterization of bivalent peptide ligands targeted to G-protein-coupled receptors. Chem.Biol., 1996, Vol.3, pages 537 to 542, ISSN 1074-5521, abstract, page 538, Figs. 1, 3, 5 | 1-11 |

Form PCT/ISA/210 (continuation of second sheet) (April 2005)

INTERNATIONAL SEARCH REPORT

International application No.
PCT/JP2005/021981

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-531568 W (Gotto-Ejin ebi), 28 October, 2003 (28.10.03), Claim 10; Par. No. [0035]; sequence listing, Sequence Number 6<br>& WO 01/02431 A1 & AU 200060401 A<br>& EP 1196435 A1 & CN 1359391 A<br>& ZA 200200092 A & KR 2002092886 A<br>& AU 763733 B | 1-11 |
| Y | JP 2004-504062 W (Gotto-Ejin ebi), 12 February, 2004 (12.02.04), Par. No. [0143]; sequence listing, Sequence Number 5<br>& WO 02/08263 A2 & AU 200170879 A<br>& EP 1301613 A2 & NO 200300239 A<br>& CZ 200300152 A3 & BR 200112634 A<br>& US 2004/0132007 A1 & NZ 524251 A<br>& MX 2003000449 A1 | 1-11 |
| A | CHAN, D.C. & KIM, P.S. HIV entry and its inhibition. Cell, 1998, Vol.93, page 681 to 684, ISSN 0092-8764 | 1-11 |
| A | Yoshie MATSUDA, "HIV no Saibo eno Shinnyu -Maku Yugo to Coiled-Coil", Igaku no Ayumi, 26 June, 1999 (26.06.99), Vol.189, No.13, ISSN 0039-2359 | 1-11 |
| P,X | Jun TAKAHASHI et al., "Hosenkin Yurai Ko-HIV Tanpakushitsu actinohivin no Ko Kassei Hen'itai no Chosei", Nippon Yakugakukai Nenkai Koen Yoshishu, 05 March, 2005 (05.03.05), Vol.125th, No.3, page 19, ISSN 0918-9823 | 1-3,5-11 |
| P,Y | TAKAHASHI, A. et al., Essential regions for antiviral activities of actinohivin, a sugar-binding anti-human immunodeficiency virus protein from an actinomycete. Arch.Biochem. Biopys., 2005, Vol.437, pages 233 to 240, ISSN 0003-9861, Tables 2, 3 | 1-11 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1152043 A
- WO 0052043 A


### Non-patent literature cited in the description


- Molecular Cloning. Cold Spring Harbor Laboratory Press, 2001
- **HORTON, R.M. ; HUNT, H.D. ; HO, S.N. ; PULLEN, J.K. ; PEASE, L.R.** Engineering hybrid genes without the use of restriction enzymes: gene splicing by overlap extension. *Gene,* 1989, vol. 77, 61-68

```
SEQUENCE LISTING

<110>  KITASATO ACADEMY

<120>  Anti-HIV Drug, Polypeptide Constituting the Same, Gene Encoding the Polypeptide and Production
Method of the Anti-HIV Drug

<130>  KUF-5833PCT

<150> JP 2004-346644
<151> 2004-11-30

<160>  12

<210>  1
<211>  114
<212>  PRT
<213>  Actynomyces K97-0003 Strain

<400>  4
Ala Ser Val Thr Ile Arg Asn Ala Gln Thr Gly Arg Leu Leu Asp Ser
 1               5                  10                  15
Asn Tyr Asn Gly Asn Val Tyr Thr Leu Pro Ala Asn Gly Gly Asn Tyr
            20                  25                  30
Gln Arg Trp Thr Gly Pro Gly Asp Gly Thr Val Arg Asn Ala Gln Thr
        35                  40                  45
Gly Arg Cys Leu Asp Ser Asn Tyr Asp Gly Ala Val Tyr Thr Leu Pro
    50                  55                  60
Cys Asn Gly Gly Ser Tyr Gln Lys Trp Leu Phe Tyr Ser Asn Gly Tyr
65                  70                  75                  80
Ile Gln Asn Val Glu Thr Gly Arg Val Leu Asp Ser Asn Tyr Asn Gly
                85                  90                  95
Asn Val Tyr Thr Leu Pro Ala Asn Gly Gly Asn Tyr Gln Lys Trp Tyr
            100                 105                 110
Thr Gly
    114


<210>  2
<211>  13
<212>  PRT
<213>  Artificial sequence
<223>  Linker sequence

<400>  2
Ala Lys Lys Leu Asn Asp Ala Gln Ala Pro Lys Ser Asp
 1               5                  10          13


<210>  3
<211>  241
<212>  PRT
<213>  Artificial sequence
<223>  Actinohivin dimer

<400>  3
Ala Ser Val Thr Ile Arg Asn Ala Gln Thr Gly Arg Leu Leu Asp Ser
 1               5                  10                  15
Asn Tyr Asn Gly Asn Val Tyr Thr Leu Pro Ala Asn Gly Gly Asn Tyr
            20                  25                  30
Gln Arg Trp Thr Gly Pro Gly Asp Gly Thr Val Arg Asn Ala Gln Thr
        35                  40                  45
Gly Arg Cys Leu Asp Ser Asn Tyr Asp Gly Ala Val Tyr Thr Leu Pro
    50                  55                  60
Cys Asn Gly Gly Ser Tyr Gln Lys Trp Leu Phe Tyr Ser Asn Gly Tyr
65                  70                  75                  80
Ile Gln Asn Val Glu Thr Gly Arg Val Leu Asp Ser Asn Tyr Asn Gly
                85                  90                  95
Asn Val Tyr Thr Leu Pro Ala Asn Gly Gly Asn Tyr Gln Lys Trp Tyr
            100                 105                 110
Thr Gly Ala Lys Lys Leu Asn Asp Ala Gln Ala Pro Lys Ser Asp Ala
        115                 120                 125
Ser Val Thr Ile Arg Asn Ala Gln Thr Gly Arg Leu Leu Asp Ser Asn
    130                 135                 140
Tyr Asn Gly Asn Val Tyr Thr Leu Pro Ala Asn Gly Gly Asn Tyr Gln
145                 150                 155                 160
```

```
Arg Trp Thr Gly Pro Gly Asp Gly Thr Val Arg Asn Ala Gln Thr Gly
                165                 170                 175
Arg Cys Leu Asp Ser Asn Tyr Asp Gly Ala Val Tyr Thr Leu Pro Cys
            180                 185                 190
Asn Gly Gly Ser Tyr Gln Lys Trp Leu Phe Tyr Ser Asn Gly Tyr Ile
        195                 200                 205
Gln Asn Val Glu Thr Gly Arg Val Leu Asp Ser Asn Tyr Asn Gly Asn
    210                 215                 220
Val Tyr Thr Leu Pro Ala Asn Gly Gly Asn Tyr Gln Lys Trp Tyr Thr
225                 230                 235                 240
Gly
241


<210>  4
<211>  726
<212>  DNA
<213>  Artificial sequence
<223>  DNA encoding Actinohivin dimer

<400>  4
gcctcggtga ccatccgcaa cgcccagacc ggccgcctgc tggacagcaa ctacaacggc      60
aacgtctaca cgctgcccgc caacggcggg aactaccagc ggtggaccgg ccccggcgac     120
ggcaccgtcc gcaacgccca gaccggccgc tgcctcgaca gcaactacga cggcgccgtc     180
tacacgctgc cgtgcaacgg cggtagctac cagaagtggc tgttctacag caacggctac     240
atccagaacg tcgagaccgg acgcgtgctc gacagcaact acaacggcaa cgtgtacaca     300
ctgccggcca acggcggcaa ctaccagaag tggtacaccg gcgccaagaa actgaacgac     360
gctcaggcgc cgaagagtga tgcctcggtg accatccgca acgcccagac cggccgcctg     420
ctggacagca actacaacgg caacgtctac acgctgcccg ccaacggcgg gaactaccag     480
cggtggaccg gccccggcga cggcaccgtc cgcaacgccc agaccggccg ctgcctcgac     540
agcaactacg acggcgccgt ctacacgctg ccgtgcaacg gcggtagcta ccagaagtgg     600
ctgttctaca gcaacggcta catccagaac gtcgagaccg gacgcgtgct cgacagcaac     660
tacaacggca acgtgtacac actgccggcc aacggcggca actaccagaa gtggtacacc     720
ggctga                                                                726


<210>  5
<211>  28
<212>  DNA
<213>  Artificial sequence
<223>  Primer Link-sen

<400>  5
gccaagaagc ttaacgacga cgctcagg                                         28


<210>  6
<211>  25
<212>  DNA
<213>  Artificial sequence
<223>  Primer Link-asn

<400>  6
atcactcttc ggcgcctgag cgtcg                                            25


<210>  7
<211>  19
<212>  DNA
<213>  Artificial sequence
<223>  Primer AA-1Bam

<400>  7
gggatccgcc tcggtgacc                                                   19


<210>  8
<211>  26
<212>  DNA
<213>  Artificial sequence
<223>  Primer AH-Link

<400>  8
gttaagcttc ttggcgccgg tgtacc                                           26
```

<210> 9
<211> 27
<212> DNA
<213> Artificial sequence
<223> Primer Link-dAH

<400> 9
gcgccgaaga gtgatgcctc ggtgacc 27

<210> 10
<211> 26
<212> DNA
<213> Artificial sequence
<223> Primer TGAEco

<400> 10
ggaattcagc cggtgtacca cttctg 26

<210> 11
<211> 30
<212> DNA
<213> Artificial sequence
<223> Primer AA-1 LIC

<400> 11
ggtattgagg gtcgcgcctc ggtgaccatc 30

<210> 12
<211> 30
<212> DNA
<213> Artificial sequence
<223> Primer TGA 114 LIC

<400> 12
agaggagagt tagagcctca gccggtgtac 30